# EUROPEAN PATENT APPLICATION

(11) **EP 1 918 288 A1**
(43) Date of publication of application: **07.05.2008**
(21) Application number: 07021105.7
(22) Date of filing: 29.10.2007
(51) Int. Cl.: C07D 403/10

(54) **A novel and improved process for the preparation of Irbesartan, an angiotensin-II receptor antagonist for the treatment of hypertension**

(30) Priority: 02.11.2006 IN MU18282006
(71) Applicant: Cadila Pharmaceuticals Limited, Bhat, Ahmedabad 382 210 (IN)
(72) Inventor: Modi, Indravadan Ambalai, Bhat, Ahmedabad - 382 210, Gujarat (IN); Pinjar, Haseena, Bhat, Ahmedabad - 382 210, Gujarat (IN); Gurusamy, Renugadevi, Bhat, Ahmedabad - 382 210, Gujarat (IN); Ponnaiah, Ravi, Bhat, Ahmedabad - 382 210, Gujarat (IN); Khamar, Bakulesh Khamar, Bhat, Ahmedabad - 382 210, Gujarat (IN)
(74) Representative: Towler, Philip Dean

(57) **Abstract**

2-Butyl-3-[[2'-(1*H*-tetrazol-5-yl)[1,1'-biphenyl]-4-yl]methyl]-1,3-diaza-spiro[4,4]non-1-en-4-one (Irbesartan) is prepared by reacting 1-(2'-cyanobipehnyl-4-yl)methyl)-2-n-butyl-4-spirocyclopentane-2-imidazolin-5-one with sodium azide and zinc halide, in organic solvent.

## Description

### FIELD OF THE INVENTION:

The present invention relates to a novel and improved process for the preparation of Irbesartan, an angiotensin-II receptor antagonist for the treatment of hypertension.

### BACKGROUND OF THE INVENTION:

Irbesartan is known by following chemical names:
(a) 2-Butyl-3-[[2'-(1*H*-tetrazol-5-yl)[1,1'-biphenyl]-4-yl]methyl]-1,3-diazaspiro[4,4]non-1-en-4-one
(b) 2-Butyl-3-[*p*-(o-*1H*-tetrazol-5-ylphenyl)benzyl]-1,3-diazaspiro[4,4]non-1-en-4-one
(c) 2-*n*-butyl-4-spirocyclopentane-1-[(2'-(tetrazol-5-yl)biphenyl-4-yl) methyl]-2-imidazolin-5-one.

The structural formula of Irbesartan is represented below. 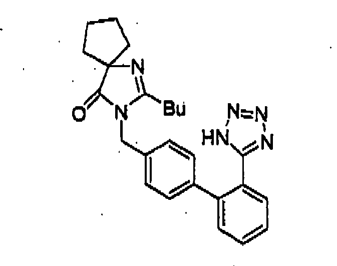 Irbesartan

The synthesis of irbesartan is first disclosed in US5270317 (equivalent EP0454511) and subsequently, several other patents disclose the synthesis of irbesartan by different methods. Basically the synthesis of this molecule involves two common intermediates namely spiroimidazole and substituted 4'-bromomethylbiphenyl.

US 5270317 describes preparation of irbesartan wherein 1-[(2'-cyanobiphenyl-4-yl)methyl]-2-n-butyl-4-spirocyclopentane-2-imidazolin -5-one which is reacted with tributyltin azide in xylene at reflux temperature for 66 hours to give a product which is isolated from the reaction mass as trityl irbesartan and then deprotected in methanol/THF mixture using 4N hydrochloric acid to get irbesartan.

US5629331 describes a process for the preparation of irbesartan from 1-[(2'-cyanobiphenyl)4-yl)methyl]-2-n-butyl-4-spirocyclopentane-2-imidazolin-5-one using sodium azide, TEA.HCl in N-methylpyrrolidone. The product is isolated from the alkaline reaction mass after acidification to pH 4.7 to 5.8 and the crude product is recrystallised from IPA/water to get Form A and ethanol/water to get Form B.

WO 2005/051943 A1 describes a process for the preparing irbesartan wherein 1-[(2'-cyanobiphenyl-4-yl)methyl]-2-n-butyl-4-spirocyclopentane-2-imidazolin-5-one is reacted with tributyltin chloride, sodium azide and TBAB in toluene at reflux temperature for 20 hours. Product is isolated from the reaction mass as trityl irbesartan and then deprotected in methanol and formic acid to get irbesartan.

WO 2006/023889 describes a method for preparing irbesartan, wherein 1-(2'-cyanobiphenyl-4-yl)methyl)-2-n-butyl-4-spirocyclopentane-2-imidazolin-5-one is reacted with sodium azide and triethylamine hydrochloride in N-methyl-2-pyrrolidone to give irbesartan.

WO 2005/113518 describes a process for preparing irbesartan wherein cyano irbesartan in xylene, is reacted with tributyltin chloride and sodium azide at reflux temperature till reaction is completed followed by aqueous work-up and recrystallization to give irbesartan.

The process involving use of zinc salt for the transformation of nitrile to tetrazole is a safe and efficient process as reported in JOC (2001) 66, 7945-50. The use of zinc salt for transforming nitrile to tetrazole has also been published in WO9637481 and US5502191.

A comprehensive review of the prior art reveals use of column chromatographic purification of intermediates, long reaction times, additional steps of protection and deprotection, processes resulting in low yields, multistep extractive workup procedures. There is a long felt need of the industry to provide a commercially scalable process of preparing irbesartan which is high yielding and devoid of disadvantages described in the prior art.

### SUMMARY OF THE INVENTION :

The main object of the present invention is to provide a process for the commercially scalable preparation of irbesartan in high yield.

Another object of the invention is to provide a process for preparing irbesartan with shorter reaction times.

Yet another object of the invention is to provide a process for preparing irbesartan which does not involve column chromatographic isolation.

Yet another object of the invention is to provide a process for preparing irbesartan avoiding multi step extractive workup.

### DETAILED DESCRIPTION OF THE INVENTION:

The present invention uses zinc halides such as zinc chloride, zinc bromide; preferably zinc chloride with sodium azide for the conversion of nitrile to tetrazole in the preparation of irbesartan.
In accordance with the present invention, synthesis of irbesartan comprises:
[1] Reacting 1-(2'-cyanobiphenyl-4-yl)methyl)-2-n-butyl-4-spiro cyclopentane-2-imidazolin-5-one with sodium azide and zinc halide in polar aprotic organic solvent followed by extractive workup to give irbesartan.

### In step [1]:

The solvent used for the reaction is polar aprotic solvent such as DMF, NMP, N, N-dimethylacetamide, tetramethyl urea, sulfolane and their like. The preferred polar aprotic solvent being DMF.
Zinc halide is selected from zinc chloride, zinc bromide, preferably zinc chloride.

The reaction mass is refluxed for about 15 hours. The product is isolated by aqueous extractive workup and the yield of irbesartan isolated is 80%.

The present invention is illustrated with following examples without limiting the scope of invention in any way.

### Example-1 Preparation of 1-(2'-cyanobiphenyl-4-yl) methyl)-2-n-butyl-4-spirocyclopentane-2-imidazolin-5-one (Cyano irbesartan) :

A suspension of 60% sodium hydride (26.4g, 0.66 mol) in DMF (150ml) was cooled to -5°C and a solution of 2-n-Butyl-4-spirocyclopentane-2-imidazolin-5-one (100g, 0.514 mol) in 250 ml DMF was added. After stirring the reaction mass for 15 minutes, a solution of 4-bromomethyl-2'-cyanobiphenyl 134g, 0.492 mol) in 600ml DMF was added over 30 minutes at -5°C. The reaction mass was stirred at 5-15°C for 1 hour, the progress of the reaction was monitored by TLC. After the completion of the reaction, 50 ml IPA was added to quench the excess of sodium hydride, concentrated to give a residue. The residue was dissolved in toluene (500ml)- water (500ml) mixture, stirred for 15 minutes and the layers were separated. The aqueous layer was extracted with toluene (100ml). The combined organic layer was acidified with dilute hydrochloric acid to adjust the pH to 3-4. Reaction mass was stirred for 3 hours at room temperature, filtered and washed with 100ml toluene. The resulting cyano irbesartan hydrochloride was basified by 5% sodium bicarbonate solution to give 160g of cyano irbesartan free base (84.4 %).

### Example-2 Preparation of 2-n-butyl-3-[(2'-(tetrazol-5-yl) biphenyl-4-yl) methyl)-1,3-diazospiro-(4,4)-non-1-ene-4-one [Irbesartan] :

To a solution of 1-(2'-cyanobiphenyl-4-yl) methyl)-2-n-butyl-4-spirocyclopentane-2-imidazolin-5-one (Cyano irbesartan) (50g, 0.129mol) in DMF was added, zinc chloride (22.12 g, 0,162mol) and sodium azide (21.10g, 0.324mol). The reaction mass was refluxed for 15 hrs and the progress of the reaction was monitored by TLC. After the reaction was over, it was cooled to room temperature and basified using 10% sodium hydroxide solution to pH 11-12. The reaction mass was stirred for 1hr and filtered. The filtrate was washed with toluene (250ml). 75 ml of IPA was added to the aqueous layer and pH was adjusted to 4-5 with con. HCl. Reaction mass was stirred for 3 hours, filtered and washed with water. The product was dried at 60°C to give 45g (81%) of irbesartan.

## Claims

1. A process for the preparation of irbesartan comprising:
Reacting 1-(2'-cyanobipehnyl-4-yl)methyl)-2-n-butyl-4-spirocyclo pentane-2-imidazolin-5-one with sodium azide and zinc halide, in organic solvent, followed by extractive workup to give irbesartan.

2. The process for the preparation of irbesartan as claimed in claim-1 wherein organic solvent used is selected from THF, N,N-dimethylacetamide, N,N-Dimethylformamide, dimethylsulfoxide, N-methyl 2-pyrrolidone, tetramethyl urea or mixtures thereof.

3. The process for the preparation of irbesartan as claimed in claim-2 wherein the preferred organic solvent is N, N-dimethylformamide.

4. The process for the preparation of irbesartan as claimed in claim-1, wherein the zinc halide is selected from zinc chloride, zinc bromide.

5. The process for the preparation of irbesartan as claimed in claim-4, wherein the preferred zinc halide is zinc chloride.

6. The process for the preparation of irbesartan as claimed in claim-1, wherein the reaction is carried out at a temperature range of 20°C to 150°C.

7. The process for the preparation of irbesartan as claimed in claim-6 wherein reaction is preferably carried out at 110°C to 140°C.
